Europäisches Patentamt

European Patent Office

Office européen des brevets

(11) Veröffentlichungsnummer: **0 514 628 A1**

# EUROPÄISCHE PATENTANMELDUNG

(21) Anmeldenummer: **92103007.8**

(22) Anmeldetag: **22.02.92**

(51) Int. Cl.5: **C07H  15/04**

(30) Priorität: **22.05.91 DE 4116669**

(43) Veröffentlichungstag der Anmeldung:
**25.11.92 Patentblatt  92/48**

(84) Benannte Vertragsstaaten:
**DE ES FR GB IT**

(71) Anmelder: **HÜLS AKTIENGESELLSCHAFT**
**Patentabteilung / PB 15 - Postfach 13 20**
**W-4370 Marl 1(DE)**

(72) Erfinder: **Ripke, Norbert, Dr.**
**Hellweg 28**
**W-4358 Haltern(DE)**

(54) **Verfahren zur Herstellung hellfarbener Alkylpolyglycoside.**

(57)  Die Herstellung von Alkylpolyglycosiden mit einer Alkylgruppe mit 8 bis 24 C-Atomen durch Umglycosidierung von $C_{1-6}$-Alkylglycosiden mit $C_{8-24}$-Alkohlen wird erfindungsgemäß in einem Verdampfer bei einer Säurezahl von 1 bis 10 mg KOH/g durchgeführt. Dabei werden farblich verbesserte Alkylpolyglycoside erhalten.

EP 0 514 628 A1

Die Erfindung betrifft ein Verfahren zur Herstellung von Alkylpolyglycosiden mit einer Alkylgruppe mit 8 bis 24 C-Atomen, bei dem Alkylglycoside mit Alkylgruppen mit 1 bis 6 C-Atomen durch Alkohole mit 8 bis 24 C-Atomen umglycosidiert werden.

Alkylpolyglycoside sind ungiftige und leicht abbaubare oberflächenaktive Stoffe. Sie werden deshalb als Wasch- und Reinigungsmittel und als Emulgatoren und Dispergatoren verwendet. Sie haben aber nur dann die gewünschten Grenzflächeneigenschaften, wenn die Alkylgruppen mindestens 8 C-Atome aufweisen.

Alkylpolyglycoside mit langkettigen Alkylresten können ganz oder teilweise aus nachwachsenden Rohstoffen hergestellt werden. Diese Alkylpolyglycoside gewinnen wegen ihrer interessanten Tensideigenschaften bei gleichzeitig sehr guter biologischer Abbaubarkeit zunehmend an Bedeutung. Für Anwendungen im Haushalt und im Kosmetikbereich müssen diese Produkte hohen ästhetischen Ansprüchen genügen. Man ist daher an Verfahren interessiert, nach denen Alkylpolyglycoside in transparenten, möglichst farblosen wäßrigen Lösungen hergestellt werden können.

Alkylpolyglycoside mit langkettigen Alkylgruppen werden im allgemeinen aus Sacchariden und Alkoholen durch Glycosidierung und Umglycosidierung hergestellt. Bei einem zweistufigen Verfahren wird beispielsweise zunächst durch Glycosidierung mit n-Butanol ein n-Butylglycosid und daraus durch Umglycosidierung mit einem langkettigen Alkohol das gewünschte langkettige Alkylpolyglycosid hergestellt. Die Produkte, die man dabei erhält, sind jedoch dunkel gefärbt.

Im EP 0 306 652 werden Alkylpolyglycoside in 2 Stufen durch Glycosidierung und Umglycosidierung hergestellt. Farblich gute Produkte erhält man hier durch Zusatz von Komplexierungs- und Bleichmitteln.

Nach EP 0 077 167 können bei der Umsetzung von Alkoholen mit Aldosen oder Ketosen Reduktionsmittel, wie hypophosphorige Säure oder schwefelige Säure, zugesetzt werden. Diese Additive sind praktisch an jeder Stelle im Prozeß von Vorteil. Durch sie wird die Farbe der Alkylglycoside verbessert.

Es sind auch vorbeugende Maßnahmen bekannt. So erhält man nach EP 0 102 558 farblich verbesserte $C_3$- bis $C_5$-Alkylglucoside, wenn man die Glucosidierung in Gegenwart eines Alkalisalzes einer Borsäure durchführt.

Nach EP 0 165 721 kann die Farbe der Produkte durch mehrstufige Bleichung mit Wasserstoffperoxid verbessert und durch Zusatz von Schwefeldioxid freisetzenden Verbindungen stabilisiert werden.

Bei allen festen und flüssigen Farbverbesserern ist es schwierig, diese Mittel nach beendeter Reaktion quantitativ zu entfernen.

In EP 0 092 876 wird das Produkt nach beendeter Umglycosidierung und Neutralisation dadurch gereinigt, daß man die überschüssigen langkettigen Alkohole mit 12 bis 18 C-Atomen in einem Dünnschichtverdampfer abdestilliert. Bei diesem nachträglichen Reinigungsverfahren wird jedoch eine Farbstoffbildung während der Glycosidierung oder der Umglycosidierung nicht unterbunden.

Es bestand daher die Aufgabe, die genannten Nachteile des Standes der Technik zuvermeiden. Bei der Herstellung von hellfarbenen Alkylpolyglycosiden sollte eine Farbstoffbildung bereits während der Umglycosidierung möglichst ohne fremde Hilfsstoffe, die später wieder abgetrennt werden müßten, unterdrückt werden.

Die Aufgabe wird erfindungsgemäß dadurch gelöst, daß man die Umglycosidierung in einem Verdampfer bei einer Säurezahl (SZ) von 1 bis 10 mg KOH/g durchführt.

Es zeigte sich überraschend, daß eine schnelle Abtrennung der bei der Umglycosidierung freiwerdenden und der gegebenenfalls zur Umglycosidierung eingeführten kurzkettigen Alkohole erheblich zur Farbverbesserung der Alkylpolyglycoside beiträgt.

Geeignete Verdampfer für das erfindungsgemäße Verfahren sind beispielsweise Rohr-, Rohrbündel-, Fallfilm- und Dünnschichtverdampfer. Dabei werden Fallfilm- und Dünnschichtverdampfer vorzugsweise eingesetzt. Reaktionsmischungen können in diesen Verdampfern einer produktschonenden Flash-Verdampfung unterworfen werden.

Die hier eingesetzten Alkylglycoside werden im allgemeinen aus einem Saccharid und einem kurzkettigen Alkohol mit 1 bis 6 C-Atomen durch säurekatalysierte Glycosidierung hergestellt.

Der Zuckeranteil der eingesetzten Alkylglycoside kann dabei von einem Monosaccharid, wie Glucose, Mannose, Gulose, Galaktose oder Talose, aber auch von einem Di- oder einem Oligosaccharid hergeleitet werden. Die Saccharideeinheiten können 1,2-, 1,3-, 1,4- oder 1,6-verknüpft sein. Es können $\alpha$- oder $\beta$-Verknüpfungen vorliegen. Die Ketten können linear oder verzweigt sein. Vorzugsweise wird Glucose bei des Glycosidierung eingesetzt.

Der Alkylanteil der eingesetzten Alkylglycoside stammt von kurzkettigen Alkoholen, wie beispielsweise Ethanol, Propanol, Butanol, Pentanol oder Hexanol. Auch Glykole, wie Ethylenglykol und Propylenglykol, können bei der Glycosidierung verwendet werden. Vorzugsweise enthalten die Alkylglycoside Butyl- oder Pentylgruppen.

Die Alkylglycoside können auch in geringem Umfange oligomerisiert sein. So werden auch Produkte mit Oligomerisationsgraden von 1 bis 3 im Sinne dieser Erfindung als Alkylglycoside angesehen.

Die Alkylglycoside kann man beispielsweise in einem Rührkessel, in einem Verdampfer oder einer Kolonne herstellen.

Die Alkylglycoside werden vorzugsweise zusammen mit einem Alkohol mit 1 bis 6 C-Atomen eingesetzt. Geeignete Alkohole sind beispielsweise Methanol, Ethanol, Butanol, Pentanol und Hexanol, aber auch Ethylenglykol und Propylenglykol. Im besonderen werden dabei bis zu 5 Gewichtsteile Alkohol mit 1 bis 6 C-Atomen, bezogen auf das Alkylglycosid, mit eingesetzt.

In einer sehr bevorzugten Ausführungsform wird Butylglucosid zusammen mit n-Butanol für die Umglycosidierung eingesetzt.

Für die Umglycosidierung geeignete langkettige Alkohole sind beispielsweise Octanol, Nonanol, Decanol, Dodecanol, Tetradecanol oder Stearylalkohol. Vorzugsweise setzt man Alkohole mit 10 bis 18 C-Atomen ein. Die Alkohole können linear sein. Sie können aber auch Verzweigungen enthalten. Man kann natürliche oder synthetische Alkohole oder Alkoholgemische einsetzen.

Als saure Katalysatoren kann man Mineralsäuren, wie Schwefel- oder Phosphorsäure, einsetzen. Gut geeignet sind auch organische Säuren, wie beispielsweise Aryl-, Alkyl- oder Aralkylsulfonsäuren.

Während der Umglycosidierung liegt die SZ vorzugsweise im Bereich von 1,5 bis 5 mg KOH/g.

Die Reaktion wird vorzugsweise bei einer Produkttemperatur von 60 bis 160 °C durchgeführt. Dabei wird ein Temperaturbereich von 80 bis 120 °C besonders bevorzugt. Die Reaktion kann bei Normaldruck, bei leichtem Unterdruck und auch bei Überdruck ausgeführt werden.

Die Verdampfer werden vorzugsweise bei einer Heiztemperatur von 120 bis 180 °C gefahren. Dabei werden meist Produkttemperaturen am Verdampferausgang von 80 bis 120 °C eingestellt.

Die hergestellten Alkylpolyglycoside weisen einen mittleren Polymerisationsgrad von 1 bis 10 auf. Dabei werden niedrige mittlere Polymerisationsgrade von 1,3 bis 5 bevorzugt.

Das erfindungsgemäße Verfahren ist produktschonend und hat folgende Vorteile:

- Die kurzkettigen Alkohole werden schnell abgetrennt. Die Umglycosidierung wird dadurch beschleunigt. Die Raum-Zeit-Ausbeute wird erhöht.
- Das Produkt wird thermisch weniger belastet. Produktschädigungen durch saure Hydrolyse werden erheblich reduziert. Man erhält deshalb farblich verbesserte Alkylpolyglycoside. Die Produkte sind hellfarben, d. h. ihre 50%igen wäßrigen Lösungen weisen Iodfarbzahlen (IFZ) von unter 25 auf.

Obwohl das Reaktionsgemisch der Umglycosidierung nicht neutralisiert ist, kommt es im Verdampfer nicht zu Anbackungen oder Verstopfungen. Das ist überraschend, da die Reaktionsmischung bei der Umglycosidierung wegen der z. T. erheblichen Löslichkeitsunterschiede und den in einigen Fällen nicht geringen Gehalten an polymeren Glycosiden nicht homogen ist.

Im allgemeinen wird bei der Durchführung des Verfahrens so vorgegangen, daß man die für die Umglycosidierung frisch bereitete Mischung direkt auf einen als Verdampfer arbeitenden Wärmetauscher gibt. Die Reaktionsmischung wird dann 5- bis 20mal über den Wärmetauscher umgewälzt. Dadurch wird innerhalb kürzester Zeit der kurzkettige Alkohol, der bei der Umglycosidierung freigesetzt wird, und der, der gegebenenfalls mit eingesetzt wurde, effizient abgetrennt.

Die folgenden Beispiele sollen die Erfindung verdeutlichen.

Beispiel 1

In einem 7-l-Rührkessel mit Thermometer und Vigreux-Kolonne werden 0,8 kg Alkylpolyglycosid in 4 l Gemisch auf 70 % Dodecanol und 30 % Tetradecanol (ALFOL[R] 1214 S, Fa. Condea, D-2212 Brunsbüttel) bei 118 °C und 30 mbar vorgelegt. Das Gemisch wird nun mit einem Volumenstrom von 40 l/h durch einen Fallfilmverdampfer (0,2 m² Fläche, 170 °C Öltemperatur) gepumpt und in den Rührkessel zurückgeleitet. Zu dem umgepumpten Produkt werden vor dem Eintritt in den Verdampfer pro Stunde 8 000 ml Gemisch aus 70 % Dodecanol und 30 % Tetradecanol, 1 200 g Butylglycosid in 2 800 ml Butanol und 30 g $H_2SO_4$ über ein Mischelement zugegeben.

Die Produkttemperatur beträgt am Ausgang des Verdampfers 119 °C. Die Säurezahl beträgt im Rührkessel ca. 3 mg KOH/g. Butylglycosid wird dabei durch das langkettige Alkoholgemisch zu Alkylpolyglycosid umglycosidiert, während Butanol abdestilliert wird.

Danach wird die kontinuierlich anfallende Reaktionsmischung neutralisiert und der überschüssige Alkohol destillativ entfernt. Der erhaltene Feststoff wird in Wasser gelöst und mit Ozon gebleicht.

Man erhält ein hellfarbenes Alkylpolyglycosid.
Mittlerer Polymerisationsgrad: 1,4
IFZ: 3 bis 5 (aus einer 50%igen wäßrigen Lösung)

Vergleichsbeispiel A

Es wird wie in Beispiel 1 verfahren. Die Umglycosidierung erfolgt jedoch in einem 10-l-Rührkessel in 3 Stunden bei 118 °C, wobei aus dem gerührten Ansatz heraus destilliert wird.

Die Aufarbeitung erfolgt wie in Beispiel 1. Dabei erhält man ein Alkylpolyglycosid mit folgende Eigenschaften:
Mittlerer Polymerisationsgrad: 1,7
IFZ: 30 (aus einer 50%igen wäßrigen Lösung)

**Patentansprüche**

1.    Verfahren zur Herstellung von Alkylpolyglycosiden mit einer Alkylgruppe mit 8 bis 24 C-Atomen durch Umglycosidierung von Alkylglycosiden mit Alkylgruppen mit 1 bis 6 C-Atomen mit Alkoholen mit 8 bis 24 C-Atomen,
dadurch gekennzeichnet,
daß man die Reaktion in einem Verdampfer bei einer Säurezahl von 1 bis 10 mg KOH/g durchführt.

2.    Verfahren nach Anspruch 1,
dadurch gekennzeichnet,
daß man die Reaktion in einem Fallfilm- oder Dünnschichtverdampfer durchführt.

3.    Verfahren nach Anspruch 1,
dadurch gekennzeichnet,
daß eine Säurezahl von 1,5 bis 5 mg KOH/g eingestellt wird.

4.    Verfahren nach Anspruch 1,
dadurch gekennzeichnet,
daß die Reaktion in einem auf 120 bis 180 °C aufgeheizten Verdampfer durchgeführt wird.

5.    Verfahren nach Anspruch 1,
dadurch gekennzeichnet,
daß die Produkttemperatur während der Reaktion 60 bis 160 °C beträgt.

6.    Verfahren nach Anspruch 5,
dadurch gekennzeichnet,
daß die Produkttemperatur 80 bis 120 °C beträgt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 092 355 (A.E. STAYLEY MANUFACTURING COMPANY) <br> * Seite 12, Zeile 1 - Zeile 12; Beispiel 1 * | 1-6 | C07H15/04 |
| X | EP-A-0 092 875 (THE PROCTER AND GAMBLE COMPANY) <br> * Seite 4, Zeile 24 - Zeile 36; Beispiel VII * | 1-6 | |
| D,X | EP-A-0 092 876 (THE PROCTER AND GAMBLE COMPANY) <br> * Seite 4, Zeile 24 - Zeile 36; Beispiel VII * | 1-6 | |
| A | EP-A-0 253 996 (HULS AKTIENGESELLSCHAFT) <br> * Ansprüche * | 1-6 | |
| | ----- | | **RECHERCHIERTE SACHGEBIETE (Int. Cl.5)** <br><br> C07H |

Der vorliegende Recherchenbericht wurde für alle Patentansprüche erstellt

| Recherchenort | Abschlußdatum der Recherche | Prüfer |
|---|---|---|
| DEN HAAG | 27 AUGUST 1992 | DAY G.J. |